Europäisches Patentamt

European Patent Office

Office européen des brevets

(11) EP 0 993 823 A1

(12) # DEMANDE DE BREVET EUROPEEN

(43) Date de publication:
**19.04.2000 Bulletin 2000/16**

(51) Int Cl.7: **A61K 7/48**, A61K 7/02

(21) Numéro de dépôt: **99401797.8**

(22) Date de dépôt: **16.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **11.09.1998 FR 9811361**

(71) Demandeur: **L'OREAL**
**75008 Paris (FR)**

(72) Inventeurs:
• **Ferrari, Véronique**
  **94700 Maisons-Alfort (FR)**
• **de la Poterie, Valérie**
  **77820 Le Chatelet-en-Brie (FR)**

(74) Mandataire: **Lhoste, Catherine**
**L'OREAL-DPI**
**6 rue Bertrand Sincholle**
**92585 Clichy Cédex (FR)**

(54) **Composition aqueuse comprenant un polyester sulfoné et un polymère radicalaire**

(57) L'invention a pour objet une composition cosmétique ou dermatologique comprenant dans une phase aqueuse:

i) un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \qquad (I)$$

dans laquelle

-   A représente un groupement 1,4-phénylène, sulfo-1,3-phénylène,
-   n va de 1 à 4,

-   au moins 35 % en mole desdits motifs de formule (I) étant des motifs de formule (I) pour lesquels A représente un groupement 1,4-phénylène et n est égal à 1,
-   au moins 7 % en mole desdits motifs de formule (I) étant des motifs de formule (I) pour lesquels A représente un groupement sulfo-1,3-phénylène,

la masse moléculaire en poids dudit oligomère copolyester étant inférieure à 20 000,
ii) au moins un système polymèrique filmogène contenant des particules de polymère radicalaire dispersées dans la phase aqueuse.

Cette composition est destinée au maquillage des matières kératiniques.

EP 0 993 823 A1

**Description**

La présente invention concerne une nouvelle composition filmogène, notamment topique, comprenant un agent gélifiant hydrophile particulier de type polyester sulfoné et un polymère radicalaire filmogène destinée en particulier aux domaines cosmétique et/ou dermatologique. L'invention se rapporte aussi à l'utilisation de cette composition pour le maquillage ou le traitement cosmétique des matières kératiniques d'être humain telles que la peau, les ongles, les cils, les sourcils, les cheveux, ou des muqueuses telles que les lèvres. Cette composition peut se présenter sous forme de gel, de pâte ou de produit coulé, notamment de stick.

Les produits de maquillage ou de soin de la peau ou des lèvres comme les fonds de teints ou les rouges à lèvres contiennent généralement des phases grasses telles que des cires et des huiles, des pigments et/ou des charges et, éventuellement des additifs comme des actifs cosmétiques ou dermatologiques. Elles peuvent contenir des gélifiants permettant d'obtenir la consistance souhaitée pour la composition selon son application.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. En outre, l'apparition de ces traces inacceptables notamment sur les cols de chemisier peut écarter certaines femmes de l'utilisation de ce type de maquillage. Il est connu de la demande EP-A-602905 d'associer des huiles volatiles à des cires pour obtenir des produits ayant des propriétés de sans-transfert améliorées. Toutefois, ces produits glissent lors de leur application sur les matières kératiniques, comme par exemple les lèvres, rendant leur application difficile à maîtriser. De plus, ces produits laissent une impression de tiraillement pendant et après leur application sur les lèvres.

Par ailleurs, les compositions après l'application sur la peau ou les lèvres peuvent s'éliminer au contact de l'eau, notamment lors de la baignade ou au contact de la pluie ou des larmes. Il est donc souhaitable de disposer de compositions présentant de bonnes propriétés de rémanence à l'eau.

Le but de la présente invention est de proposer une nouvelle composition filmogène aqueuse ne présentant pas les inconvénients évoqués ci-dessus et présentant notamment des propriétés de sans transfert total, de rémanence à l'eau, d'absence de tiraillement pendant et après l'application sur les matières kératiniques, et d'application facile.

Les inventeurs ont découvert qu'une telle composition pouvait être obtenue en utilisant un agent gélifiant hydrophile particulier de type polyester sulfoné et un système polymérique contenant un polymère radicalaire. On obtient alors une composition laissant après l'application un film présentant une bonne tenue, ne transférant pas du tout, et résistant à l'eau. Le film est notamment brillant, non collant et confortable (non tiraillement) pendant et après l'application de la composition sur les matières kératiniques. Le film produit également une sensation de fraîcheur et est facile à démaquiller avec les démaquillants habituellement utilisés. De plus, la composition est moins glissante que les produits sans transfert contenant des huiles volatiles, facilitant ainsi son application sur les matières kératiniques.

La présente invention a donc pour objet une nouvelle composition filmogène comprenant dans une phase aqueuse un agent gélifiant hydrophile de type polyester sulfoné, caractérisée par le fait que l'agent gélifiant hydrophile est un oligomère copolyester téréphtalique hydrosoluble ou hydrodispersible et que la composition comprend également un système polymérique contenant des particules de polymère radicalaire dispersées dans la phase aqueuse.

a) L'oligomère copolyester téréphtalique :

Les agents gélifiants particuliers utilisés selon l'invention sont des oligomères copolyesters téréphtaliques hydrosolubles ou hydrodispersables comprenant essentiellement des motifs répétitifs dicarboxylates de formule (I):

$$[-CO-A-CO-O-(CH_2-CH_2O)_n-] \qquad (I)$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids desdits oligomères copolyesters étant inférieure à 20 000, de préférence inférieure à 15 000.

De manière préférentielle, au moins 40 % en mole, et plus préférentiellement entre 40 et 90 % en mole des motifs de formule (I) sont des motifs de formule (I) pour lesquels A représente un groupement 1,4-phénylène et n est égal à 1.

De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO\text{-}A\text{-}CO\text{-}O\text{-}(CH_2\text{-}CH_2\text{-}O)_n\text{-}H \hspace{4cm} (I')$$

dans laquelle A et n sont définis ci-dessus.

Lesdits oligomères peuvent également présenter en extrêmités de chaîne, et en quantité mineure, des grou-pements de formules

$$-A\text{-}CO\text{-}OH$$

$$-A\text{-}CO\text{-}OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment de sodium ou de potassium, ou d'un sulfonate d'ammonium ou de mono-, di-, tri- ou tétra-alkylammonium inférieur. Par alkylammonium inférieur, on entend de préférence un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence de 0,5 à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids allant de 5 000 à 14 000, et plus préférentiellement de 8 000 à 10 000.

Les masses moléculaires en poids sont mesurées par chromatographie par perméation de gel dans le diméthylacé-tamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

- d'estérification de diacides et de diols et polycondensation
- de transestérification de diesters et de diols et polycondensation
- d'autocondensation d'hydroxyacides
- de Schotten-Baumann par mise en oeuvre de diols et de chlorures d'acide et polycondensation
- de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les oligomères copolyesters téréphtaliques utilisés selon l'invention peuvent être préparés par estérification et/ou transestérification / polycondensation d'une composition monomère à base:

- d'acide, anhydride ou diester téréphtalique (Tp)
- d'acide, anhydride ou diester sulfoisophtalique (Slp)
- éventuellement d'acide, anhydride ou diester isophtalique (Ip)
- et d'éthylène glycol (EG)

selon des quantités relatives correspondant à

\* un rapport molaire (Slp) / [(Tp)+(Slp)+(Ip)] d'au moins 7/100, de préférence d'au moins 10/100, tout particulièrement de 10/100 à 25/100

\* un rapport molaire (Ip) / [(Tp)+(Slp)+(Ip)] de 20/100 au plus, de préférence de 5/100 au plus

\* un rapport molaire (EG) / [(Tp)+(Slp)+(Ip)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (Slp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation. Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification. L'opération est réalisée en présence des mêmes types de catalyseur et de limiteur d'éthérification que ceux mis en oeuvre lors de l'opération de transestérification, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997.

L'oligomère copolyester téréphtalique peut être présent dans la composition selon l'invention en une teneur allant de 0,5 % à 40 % en poids, par rapport au poids total de la composition. Selon la forme recherchée, gélifiée, pâteuse ou solide, on emploiera des quantités différentes de gélifiant particulier. Pour une composition solide, on emploiera de préférence de 10 à 40 % en poids de gélifiant particulier, plus préférentiellement de 15 à 30 % en poids. Pour une composition gélifiée ou pâteuse, on emploiera de préférence de 0,5 à 10 % en poids de gélifiant particulier, plus préférentiellement de 2 à 5 % en poids.

b) Le système polymérique :

Le système polymérique contenant le polymère radicalaire en dispersion aqueuse, présent dans la composition selon l'invention, doit conduire seul à la formation d'un film ayant une dureté allant de 5 à 90 secondes, de préférence de 15 à 60, et mieux de 20 à 60 pour que la composition selon l'invention conduise à un film ayant à la fois des propriétés de sans transfert et de résistance à l'eau.

La dureté du film de polymère est mesurée sur un film obtenu après séchage, durant 24 heures à 30 °C et à 50 % d'humidité relative, d'une couche de 300 μm d'épaisseur (avant séchage) d'une dispersion aqueuse à 28 % de matière sèche desdites particules de polymère radicalaire. La dureté du film est mesurée selon la norme ASTM D-43-66, ou la norme NF-T 30-016 (octobre 1981), à l'aide d'un pendule de Persoz.

On a constaté que les systèmes polymériques contenant le polymère radicalaire en dispersion aqueuse dont le film présente une dureté allant de 20 à 60 secondes, comme définie précédemment, conviennent particulièrement bien lorsque le composition se présente sous la forme solide, notamment lorsque la teneur en oligomère copolyester téréphtalique va de 10 à 40 % en poids, par rapport au poids total de la composition. En effet, ces systèmes polymériques permettent d'obtenir une composition sous forme solide, par exemple en stick ou en coupelle, sans diminution de la dureté du stick ou de la coupelle par rapport à une composition solide analogue ne contenant que l'oligomère copolyester téréphtalique. La composition solide selon l'invention permet également, après son application, la formation d'un film sur les matières kératiniques ayant de bonnes propriétés de sans-transfert et de résistance à l'eau.

On entend par système polymérique filmogène un système comprenant un polymère conduisant à la formation d'un film isolable.

Par polymère radicalaire, on entend un polymère obtenu par polymérisation de monomères à insaturation notamment éthylénique, chaque monomère étant susceptible de s'homopolymériser (à l'inverse des polycondensats). Les polymères de type radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

On utilise de préférence des polymères radicalaires anioniques, c'est-à-dire des monomères ayant au moins un monomère à groupement acide.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C20, de préférence en C1-C8, des (méth)acrylates d'aryle, en particulier d'aryle en C6-C10, des (méth)acrylates d'hydroxyalkyle, en particulier d'hydroxyalkyle en C2-C6 .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La liste des monomères donnée n'est pas limitative et il est possible d'utiliser tout monomère connu de l'homme du métier dans les catégories de monomères acryliques et vinyliques.

Selon l'invention, on utilise de préférence comme polymère filmogène un copolymère choisi parmi les copolymères acide (méth)acrylique / (méth)acrylate, acide (méth)acrylique / (méth)acrylate / styrène, acide (méth)acrylique/ styrène, (méth)acrylate/styrène, les copolymères de (méth)acrylates.

Lorsque le polymère utilisé selon l'invention comprend des monomères porteur de goupement salifiable (par exemple un groupe acide carboxylique), il peut être neutralisé, totalement ou en partie à l'aide d'un agent neutralisant (en l'occurrence une base pour neutraliser le groupement acide) bien connu de l'homme du métier.

La neutralisation peut, en outre, favoriser la mise en dispersion, notamment dans l'eau, du polymère, voire même stabiliser ladite dispersion.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur base de ses connaissances générales.

La teneur en matière sèche desdites dispersions aqueuses selon la présente invention peut être de l'ordre de 5-60% en poids, et de préférence 30-50%, par rapport au poids total de la dispersion.

La taille des particules de polymère radicalaire en dispersion aqueuse peut aller de 2 à 300 nm, et de préférence de 20 à 150 nm.

Selon un premier mode de réalisation de la composition selon l'invention, le système polymérique consiste essentiellement en un ou plusieurs polymère(s) filmogène(s) radicalaire(s) ayant la caractéristique de dureté décrite précédemment.

Lorsque le polymère filmogène radicalaire ne permet pas d'obtenir seul un film ayant la dureté mentionnée précédemment, il est possible d'ajouter un composé dont la fonction est de modifier les propriétés du polymère filmogène pour obtenir le système polymérique souhaité. Aussi, selon un second mode de réalisation de la composition selon l'invention, on peut ajouter au polymère filmogène radicalaire au moins un agent auxiliaire de filmification permettant d'obtenir un film de polymère ayant la dureté telle que décrite précédemment. L'agent auxiliaire de filmification permet notamment d'obtenir un film de composition sur les matières kératiniques bien résistant à l'eau.

Dans ce cas, le système polymérique comprend un mélange d'un ou plusieurs polymères filmogènes radicalaires et d'au moins un agent auxiliaire de filmification.

Un tel agent auxiliaire de filmification peut être choisi parmi tous les composés connus de l'homme du métier comme étant susceptibles de remplir la fonction recherchée, et être notamment choisi parmi les agents plastifiants. En outre, lorsque le système polymérique selon l'invention comprend au moins une dispersion aqueuse de particules de polymère filmogène, l'agent auxiliaire de filmification peut aussi être choisi parmi les agents de coalescence. Cet agent auxiliaire peut être hydrosoluble ou insoluble dans l'eau et peut éventuellement se présenter sous forme de dispersion aqueuse.

La quantité d'agent auxiliaire de filmification peut être choisie par l'homme du métier sur base de ses connaissances générales, de manière à obtenir un système polymérique conduisant à un film ayant la dureté souhaitée, tout en conservant à la composition des propriétés cosmétiquement et/ou dermatologiquement acceptables. Cette quantité peut notamment aller de 0 à 25 % en poids, par rapport au poids total de la composition, et mieux de 0 à 15 % en poids.

Comme système polymérique utilisable dans la composition selon l'invention, on peut par exemple citer les dispersions aqueuses de polymère acrylique vendues sous les dénominations "NEOCRYL A 523", "NEOCRYL A 1070", "NEOCRYL A 1090", "NEOCRYL BT24" par la société ZENECA, "DYFLEX LP9419" par la société DYFLEX.

Selon l'invention, le polymère radicalaire peut être présent en matières sèches dans la composition en une quantité allant de 2 % à 40 % en poids par rapport au poids total de la composition, et de préférence de 5 % à 15 % en poids.

Avantageusement, l'oligomère copolyester téréphtalique et le polymère radicalaire peuvent être présents dans la composition selon l'invention dans un rapport pondéral oligomère/polymère radicalaire allant de 0,5 à 20, et de préférence de 1 à 5.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les agents anti-UV, les conservateurs, les agents épaississants, les tensioactifs, les cires, les huiles, les parfums, les agents modificateurs de pH, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être utilisée pour le maquillage, le soin ou le traitement cosmétique non thérapeutique des matières kératiniques et/ou des muqueuses. La composition de maquillage peut être un produit de maquillage des lèvres, notamment sous forme de pâte ou de stick, un fond de teint, un fard à paupières ou à joue, un mascara pour les cils ou les cheveux, un eye-liner, un anti-cernes, ou bien encore une composition de maquillage du corps. La composition de traitement ou de soin cosmétique peut être une composition pour le soin du visage, du cou, des mains, du corps, des ongles ou des lèvres, une composition déodorante ou encore de protection solaire.

L'invention se rapporte également à un procédé non thérapeutique de traitement cosmétique ou de maquillage des matières kératiniques et/ou des muqueuses consistant à appliquer sur les matières kératiniques et/ou des muqueuses une composition telle que décrite précédemment.

L'invention a également pour objet l'utilisation d'un oligomère copolyester téréphtalique et d'un système polymérique tels que définis précédemment, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

L'invention a aussi pour objet l'utilisation d'un oligomère copolyester téréphtalique et d'un système polymérique tels que définis précédemment dans une composition cosmétique ou dermatologique pour l'obtention d'un film de cette composition résistant à l'eau et/ou frais et/ou confortable.

L'invention a encore pour objet l'utilisation d'une composition telle que définie précédemment pour l'obtention d'un film de cette composition résistant à l'eau et/ou ayant des propriétés de non transfert et/ou frais et/ou confortable.

Un autre objet de l'invention est l'utilisation d'un système polymérique tel que défini précédemment dans une composition comprenant dans une phase aqueuse un oligomère copolyester téréphtalique tel que défini précédemment pour obtenir un produit solide stable résistant à l'eau.

On va maintenant donner des exemples illustrant la présente invention sans toutefois la limiter.

### Exemple 1    Préparation d'un oligomère copolyester téréphtalique

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit:

- 11,47 moles de téréphtalate de diméthyle
- 2,53 moles de diméthylisophtalate-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.
Pendant la période d'introduction du mélange et pendant la période de chauffage jusqu'à 250°C, on distille un mélange d'eau et d'éthylène glycol sans rétrogradation.
Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.
Le copolyester obtenu présente les caractéristiques structurales décrites au tableau 1 ci-après, dans lequel:

\* "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"SIp" signifie : motif sulfoisophtalique

Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

\* "%GT/$\Sigma$ motifs" correspond au % molaire des motifs de formule (I)

$$[-CO-A-CO-O-(CH_2-CH_2-O)_n-] \tag{I}$$

où A est 1,4 phénylène et n=1
par rapport à l'ensemble des motifs de formule (I)
où A est 1,4 phénylène, sulfo 1,3 phénylène et éventuellement 1,3 phénylène et n va de 1 à 4
"%GT/$\Sigma$ motifs" se calcule par la formule suivante :

$$\%GT/\Sigma \text{ motifs} = (\% \text{ molaire de motifs Tp}) \times (\% \text{ molaire de motifs G}) / 100$$

\* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| SIp | 15 |
| %GT/$\Sigma$ motifs | 46,5 |
| % molaire des motifs diols | |
| G | 56,8 |
| 2G | 30,7 |
| 3G | 10 |
| 4G | 2,5 |
| Mw | 8 000 |

## Exemple 2 :

On a préparé un rouge à lèvres en forme de stick ayant la composition suivante :

- oligomère copolyester téréphtalique de l'exemple 1      20 %
- dispersion aqueuse de copolymère acrylique à 60 % de matières sèches (NEOCRYL A 523 de ZENECA)      10 % MA
- pigments      5 %
- propylène glycol      2,5 %
- eau      qsp      100 %

On obtient un rouge à lèvres permettant l'obtention d'un film "sans transfert" total et qui résiste parfaitement bien à l'eau.

**Exemple 3:**

On a préparé un rouge à lèvres en forme de stick ayant la composition suivante :

- oligomère copolyester téréphtalique de l'exemple 1        20 %
- dispersion aqueuse de polymère acrylique à 45 % de matières sèches (NEOCRYL BT 24 de ZENECA)        10 % MA
- pigments        5 %
- propylène glycol        2,5 %
- eau        qsp        100 %

On obtient un rouge à lèvres permettant l'obtention d'un film "sans transfert" total et qui résiste parfaitement bien à l'eau. On a constaté également que la dureté du stick n'est pas inférieure à la dureté d'un stick similaire ne contenant pas la dispersion aqueuse de polymère acrylique.

**Exemple 4 :**

On a préparé un mascara ayant la composition suivante :

- oligomère copolyester téréphtalique de l'exemple 1        10 %
- dispersion aqueuse de polymère acrylique à 40 % de matières sèches (DYFLEX LP 9419 de DYFLEX)        10 % MA
- pigments        8 %
- propylène glycol        4 %
- eau        qsp        100 %

**Exemple 5 :**

On a préparé un produit de maquillage du corps ayant la composition suivante :

- oligomère copolyester téréphtalique de l'exemple 1        5 %
- dispersion aqueuse de polymère acrylique à 45 % de matières sèches (NEOCRYL A 1070 de ZENECA)        5 % MA
- pigments        5 %
- propylène glycol        2,5 %
- eau        qsp        100 %

**Revendications**

1. Composition comprenant dans une phase aqueuse un agent gélifiant hydrophile de type polyester sulfoné, caractérisée en ce que :

   i) le gélifiant hydrophile de type polyester sulfoné est un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

   dans laquelle

   - A représente un groupement 1,4-phénylène, sulfo-1,3-phénylène et éventuellement 1,3-phénylène,
   - n va de 1 à 4,
   - au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
ii) et que la composition comprend également au moins un système polymèrique filmogène contenant des particules de polymère radicalaire dispersées dans la phase aqueuse, le système polymérique étant apte à former un film ayant une dureté allant de 5 à 90 secondes.

2. Composition selon la revendication 1, caractérisée par le fait que l'oligomère copolyester a une masse moléculaire en poids inférieure à 15 000.

3. Composition selon la revendication 1 ou 2, caractérisée par le fait que l'oligomère copolyester téréphtalique a une masse moléculaire en poids allant de 5 000 à 14 000, et mieux de 8 000 à 10 000.

4. Composition selon l'une quelconque des revendications précédentes, caractérisée par le fait que l'oligomère copolyester téréphtalique comprend au moins 40 % en mole desdits motifs de formule (I) pour lesquels A représente un groupement 1,4-phénylène et n est égal à 1.

5. Composition selon l'une quelconque des revendications précédentes, caractérisée par le fait que l'oligomère copolyester téréphtalique comprend de 40 à 90 % en mole desdits motifs de formule (I) pour lesquels A représente un groupement 1,4-phénylène et n est égal à 1.

6. Composition selon l'une quelconque des revendications précédentes, caractérisée par le fait que l'oligomère copolyester téréphtalique comprend au moins 10 % en mole des motifs de formule (I) pour lesquels A représente un groupement sulfo-1,3-phénylène.

7. Composition selon l'une quelconque des revendications précédentes, caractérisée par le fait que l'oligomère copolyester téréphtalique comprend de 10 % et 25 % en mole desdits motifs de formule (I) pour lesquels A représente un groupement sulfo-1,3-phénylène.

8. Composition selon l'une quelconque des revendications précédentes, caractérisée par le fait que l'oligomère copolyester téréphtalique comprend jusqu'à 20 % en mole, et de préférence de 0,5 % à 5 % en mole, de motifs de formule (I) pour lesquels A représente un groupement 1,3-phénylène.

9. Composition selon l'une quelconque des revendications précédentes, caractérisée par le fait que l'oligomère copolyester téréphtalique est présent en une teneur allant de 0,5 % à 40 % en poids, par rapport au poids total de la composition.

10. Composition selon l'une quelconque des revendications précédentes sous forme solide, caractérisée par le fait que l'oligomère copolyester téréphtalique est présent en une teneur allant de 10 % à 40 % en poids, par rapport au poids total de la composition, et mieux de 15 % à 30 % en poids.

11. Composition selon l'une quelconque des revendications précédentes sous forme de gel, caractérisée par le fait que l'oligomère copolyester téréphtalique est présent en une teneur allant de 0,5 % à 10 % en poids, par rapport au poids total de la composition, et mieux de 2 % à 5 % en poids.

12. Composition selon l'une quelconque des revendications précédentes, caractérisée par le fait que la dureté du film va de 15 à 60 secondes, et mieux de 20 à 60 secondes.

13. Composition selon l'une quelconque des revendications précédentes, caractérisée par le fait que le polymère radicalaire est un polymère vinylique.

14. Composition selon l'une quelconque des revendications précédentes, caractérisée par le fait que le polymère radicalaire résulte de la polymérisation de monomères choisis dans le groupe formé par les acides carboxyliques insaturés $\alpha,\beta$-éthyléniques, les (méth)acrylates, les (méth)acrylamides, les esters vinyliques, les monomères styréniques.

**15.** Composition selon l'une des revendications précédentes, caractérisée par le fait que le polymère radicalaire est choisi dans le groupe des copolymères formé par les copolymères acide (méth)acrylique / (méth)acrylate, acide (méth)acrylique / (méth)acrylate / styrène, acide (méth)acrylique/ styrène, (méth)acrylate/styrène, les (méth) acrylates.

**16.** Composition selon l'une quelconque des revendications précédentes, caractérisée par le fait que les particules de polymère radicalaire ont une taille allant 2 à 300 nm.

**17.** Composition selon l'une quelconque des revendications précédentes, caractérisée par le fait que le polymère radicalaire est présent en une teneur en matières sèches allant de 2 % à 40 % en poids, de préférence de 5 % à 15 % en poids, par rapport au poids total de la composition.

**18.** Composition selon l'une quelconque des revendications précédentes, caractérisée par le fait que le système polymérique consiste essentiellement en un ou plusieurs polymère(s) radicalaire(s).

**19.** Composition selon l'une quelconque des revendications précédentes, caractérisée par le fait que le système polymérique comprend au moins un agent auxiliaire de filmification.

**20.** Composition selon la revendication 19, caractérisée par le fait que l'agent auxiliaire de filmification est un agent plastifiant.

**21.** Composition selon la revendication 19 ou 20, caractérisée par le fait que l'agent auxiliaire de filmification est un agent de coalescence.

**22.** Composition selon l'une quelconque des revendications précédentes, caractérisée par le fait que l'oligomère copolyester téréphtalique et le polymère radicalaire sont présents selon un rapport pondéral oligomère/polymère radicalaire allant de 0,5 à 20, et mieux de 1 à 5.

**23.** Composition selon l'une quelconque des revendications précédentes, caractérisée par le fait que la composition est une composition cosmétique ou dermatologique.

**24.** Composition selon l'une quelconque des revendications précédentes, caractérisée par le fait qu'elle comprend au moins un adjuvant choisi dans le groupe formé par les colorants, les pigments, les nacres, les agents anti-UV, les conservateurs, les agents épaississants, les agents plastifiants, les tensioactifs, les cires, les huiles, les parfums, les agents modificateurs de pH, les agents hydratants.

**25.** Composition selon l'une quelconque des revendications précédentes, caractérisée par le fait qu'elle se présente sous forme de composition de maquillage ou de traitement cosmétique des matières kératiniques et/ou des muqueuses.

**26.** Composition selon l'une quelconque des revendications précédentes, caractérisée par le fait que la composition se présente sous la forme d'un produit de maquillage des lèvres, d'un fond de teint, d'un fard à paupières ou à joue, d'un mascara, d'un eye-liner, d'un anti-cernes, d'une composition de maquillage du corps, d'une composition pour le soin du visage, du cou, des mains, du corps, des ongles ou des lèvres, d'une composition déodorante, d'une composition de protection solaire.

**27.** Procédé non thérapeutique de maquillage ou de traitement des matières kératiniques et/ou des muqueuses, caractérisé par le fait que l'on applique sur les matières kératiniques et/ou des muqueuses une composition selon l'une des revendications 1 à 26.

**28.** Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un système polymèrique filmogène contenant des particules de polymère radicalaire en dispersion aqueuse, le système polymérique étant apte à former un film ayant une dureté allant de 5 à 90 secondes,
dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

29. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et
d'un système polymèrique filmogène contenant des particules de polymère radicalaire en dispersion aqueuse, le système polymérique étant apte à former un film ayant une dureté allant de 5 à 90 secondes,
dans une composition cosmétique ou dermatologique pour l'obtention d'un film de cette composition résistant à l'eau et/ou frais et/ou confortable.

30. Utilisation d'une composition selon l'une quelconque des revendications 1 à 26 pour l'obtention d'un film de cette composition résistant à l'eau et/ou ayant des propriétés de non transfert et/ou frais et/ou confortable.

31. Utilisation d'un système polymèrique filmogène contenant des particules de polymère radicalaire en dispersion aqueuse, le système polymérique étant apte à former un film ayant une dureté allant de 5 à 90 secondes, dans une composition comprenant dans une phase aqueuse un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,

- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
pour obtenir un produit solide stable résistant à l'eau.

**Office européen
des brevets**

**RAPPORT PARTIEL
DE RECHERCHE EUROPEENNE**
qui selon la règle 45 de la Convention sur le brevet
européen est considéré, aux fins de la procédure ultérieure,
comme le rapport de la recherche européenne

Numéro de la demande

EP 99 40 1797

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | GB 2 238 242 A (MAYBE HOLDING) 29 mai 1991 (1991-05-29)<br><br>* revendications 1,3,5,9,10 *<br>* page 4, ligne 15-22 *<br>* page 5, ligne 23-26 *<br>* page 6, ligne 8 – page 7, ligne 33 *<br>* page 9, ligne 1-30 *<br>--- | 1,2,6,7, 9,11,13, 14, 17-20, 22-31 | A61K7/48 A61K7/02 |
| X | WO 96 33689 A (PROCTER & GAMBLE) 31 octobre 1996 (1996-10-31)<br><br>* revendications 1,3,5,6 *<br>* page 9, ligne 1-28 *<br>* page 10, ligne 27 – page 13, ligne 22 *<br>---<br><br>-/-- | 1,2,6,7, 9,11,13, 14, 17-20, 23-31 | |

**DOMAINES TECHNIQUES
RECHERCHES** (Int.Cl.7)

A61K

### RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 14 décembre 1999 | Peeters, J |

CATEGORIE DES DOCUMENTS CITES

X : particulièrement pertinent à lui seul
Y : particulièrement pertinent en combinaison avec un
   autre document de la même catégorie
A : arrière–plan technologique
O : divulgation non–écrite
P : document intercalaire

T : théorie ou principe à la base de l'invention
E : document de brevet antérieur, mais publié à la
   date de dépôt ou après cette date
D : cité dans la demande
L : cité pour d'autres raisons
   
& : membre de la même famille, document correspondant

EPO FORM 1503 03.82 (P04C08)

**EP 0 993 823 A1**

**Office européen**

**des brevets**

**RECHERCHE INCOMPLETE
FEUILLE SUPPLEMENTAIRE C**

Numéro de la demande

EP 99 40 1797

```
Revendications ayant fait
l'objet de recherches complètes:
     2-11,13-27,30,31

Revendications ayant fait
l'objet de recherches incomplètes:
     1,12,28,29

Raison pour la limitation de la recherche:

Les revendications 1,12,28,29 présentes ont trait à un produit/une
utilisation défini (entre autres)
au moyen des paramètres suivants:
P1: dureté allant de 5 à 90 sec.
P2: dureté allant de 15 à 60 sec.

L'utilisation de ces paramètres est considérée , dans le présent
contexte, comme menant à un manque de clarté au sens de l'Article 84 CBE.
Il est impossible de comparer les paramètres que le déposant a choisi
d'utiliser avec ce qui est révélé dans l'état de la technique. Le manque
de clarté qui en découle est tel q'une recherche significative complète
est impossible. Par conséquent, ces paramètres n'ont pas été pris en
considération pendant la recherche.
```

15

**EP 0 993 823 A1**

Office européen
des brevets

**RAPPORT PARTIEL
DE RECHERCHE EUROPEENNE**

Numero de la demande

EP 99 40 1797

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| X | US 4 300 580 A (G.J. O'NEILL, A.R. ROTHWELL) 17 novembre 1981 (1981-11-17)<br><br>* revendications 1,3,4 *<br>* colonne 1, ligne 65 – colonne 3, ligne 22 *<br>* colonne 4, ligne 39-53 *<br>* exemple 5 *<br>----- | 1,6,7,<br>9-11,13,<br>14,17,<br>18,<br>23-25,27 | |

DOMAINES TECHNIQUES
RECHERCHES (Int.Cl.7)

EPO FORM 1503 03.82 (P04C11)

16

**ANNEXE AU RAPPORT DE RECHERCHE EUROPEENNE
RELATIF A LA DEMANDE DE BREVET EUROPEEN NO.**     EP 99 40 1797

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

14-12-1999

| Document brevet cité au rapport de recherche | | Date de publication | Membre(s) de la famille de brevet(s) | | Date de publication |
|---|---|---|---|---|---|
| GB 2238242 | A | 29-05-1991 | US | 5053221 A | 01-10-1991 |
| | | | CA | 2030289 A | 21-05-1991 |
| | | | JP | 2814018 B | 22-10-1998 |
| | | | JP | 3209308 A | 12-09-1991 |
| | | | MX | 172080 B | 01-12-1993 |
| WO 9633689 | A | 31-10-1996 | AU | 5377596 A | 18-11-1996 |
| | | | CA | 2219677 A | 31-10-1996 |
| | | | CN | 1184414 A | 10-06-1998 |
| | | | CZ | 9703381 A | 18-03-1998 |
| | | | EP | 0822799 A | 11-02-1998 |
| | | | JP | 11504326 T | 20-04-1999 |
| US 4300580 | A | 17-11-1981 | AUCUN | | |

EPO FORM P0460

Pour tout renseignement concernant cette annexe : voir Journal Officiel de l'Office européen des brevets. No.12/82